# EUROPEAN PATENT SPECIFICATION

(11) **EP 2 365 948 B1**
(45) Date of publication and mention of the grant of the patent: **04.10.2017**
(21) Application number: 09810797.2
(22) Date of filing: 15.12.2009
(51) Int. Cl.: C04B 38/00, C04B 26/02, A61L 27/56

(54) **PROCESS FOR THE PREPARATION OF SELF STANDING NANOPARTICLE NETWORKS/SCAFFOLDS WITH CONTROLLABLE VOID DIMENSIONS**
VERFAHREN ZUR HERSTELLUNG VON SELBSTSTEHENDEN NANOPARTIKELNETZWERKEN/-GERÜSTEN MIT KONTROLLIERBAREN HOHLRAUMABMESSUNGEN
PROCÉDÉ DE FABRICATION DES RÉSEAUX/ÉCHAFAUDAGES DE NANOPARTICULES AUTOSTABLES À DIMENSIONS DE VIDES SÉLECTIVES

(30) Priority: 15.12.2008 IN DE28282008
(43) Date of publication of application: 21.09.2011
(73) Proprietor: Council of Scientific & Industrial Research, New Delhi 110 001 (IN)
(72) Inventor: KUMARASWAMY, Guruswamy, Pune Maharashtra 411008 (IN); SHARMA, Kamendra Prakash, Pune Maharashtra 411008 (IN)
(74) Representative: Fairbairn, Angus Chisholm
(86) International application number: PCT/IN2009/000723
(87) International publication number: WO 2010/070679

(56) References cited:
- US-A1- 2003 207 975
- US-A1- 2006 057 355
- HAJJI P ET AL: "SYNTHESIS-MORPHOLOGY-MECHANICAL PROPERTIES RELATIONSHIPS OF POLYMER-SILICA NANCOMPOSITE HYBRID MATERIALS" ORGANIC/INORGANIC HYBRID MATERIALS II. SAN FRANCISCO, CA, APRIL 5 - 9, 1999; [MATERIALS RESEARCH SOCIETY SYMPOSIUM PROCEEDINGS. VOL. 576], WARRENDALE, PA : MRS, US, vol. 576, 5 April 1999 (1999-04-05), pages 357-362, XP000897936 ISBN: 978-1-55899-483-6
- ULRICH R ET AL: "NANO-OBJECTS WITH CONTROLLED SHAPE, SIZE, AND COMPOSITION FROM BLOCK COPOLYMER MESOPHASES" ADVANCED MATERIALS, WILEY VCH VERLAG, DE LNKD- DOI:10.1002/(SICI)1521-4095(199902)11:2<14 1::AID-ADMA141>3.3.CO;2-I, vol. 11, no. 2, 22 January 1999 (1999-01-22), pages 141-146, XP000877978 ISSN: 0935-9648
- DEENDAYAL MANDAL ET AL: "Cellular uptake of gold nanoparticles directly cross-linked with carrier peptides by osteosarcoma cells" JOURNAL OF MATERIALS SCIENCE: MATERIALS IN MEDICINE, KLUWER ACADEMIC PUBLISHERS, BO, vol. 20, no. 1, 21 September 2008 (2008-09-21), pages 347-350, XP019680134 ISSN: 1573-4838

## Description

### FIELD OF THE INVENTION

The present invention relates to a process for preparing self standing network of nanoparticles/scaffolds with controllably variable mesh size.

### BACKGROUND OF THE INVENTION

Porous scaffolds, especially nanoporous to micro porous scaffolds, find a variety of areas of applications, such as catalysis, optical, electrical, electronic, electromagnetic devices, cell growth, drug delivery and chromatography amongst many others.

Reference may be made to an article titled, "Synthesis of micro-mesoporous bimodal silica nanoparticles using lyotropic mixed surfactant liquid-crystal templates", 2006, 91, 172-180 in the journal titled Microporous and mesoporous materials ISSN 1387-1811 by MORI Hiroshi; UOTA Masafumi et.al. discloses micro-mesoporous bimodal silica nanoparticles with a particle diameter of as small as 40-90 nm synthesized by a two-step reaction based on the polymerization of silicate (TEOS) species confined to the mixed surfactant hexagonal-structured liquid-crystal (LC) templates of nonaethyleneglycol dodecylether (C₁₂EO₉) and polyoxyethylene (20) sorbitan monostearate (Tween60) or eicosaethylene-glycol octadecyl ether (C₁₈EO₂₀).

Reference may be made to an article titled "Formation of highly porous biodegradable scaffolds for tissue engineering" by Antonios G. Mikos and Johnna S. Temenoff published in EJB Electronic Journal of Biotechnology ISSN: 0717-3458, Vol.3 No.2, Issue of August 15, 2000; discloses scaffold formation using different techniques, which include fiber bonding, solvent casting/particulate leaching, gas foaming and phase separation. It has been found that the various parameters which influence the pore morphology are polymer concentration, cooling method and time, solvent/non-solvent ratio, the presence of surfactants etc. Foams up to 90% porosity, with pores of approximately 100 µm, have been disclosed.

Reference may be made to patent application US 6852920, wherein a solar cell device, comprising two or more materials having different electron affinities, the solar cell device being characterized by an architecture wherein two or more materials are regularly arrayed and wherein the presence of the two or more materials alternates within distances of between about 1 nm and about 100 nm, the architecture is characterized by a mesoporous template having a conducting or semi conducting inorganic media containing pores, wherein the pores are filled with a conducting or semi conducting polymer material having a different electron affinity than the surrounding conducting or semi conducting inorganic media.

Reference may be made to an article titled "Aligned two- and three-dimensional structures by directional freezing of polymers and nanoparticles" by Haifei Zhang et. al. published on 25 September 2005, in Nature Materials 4, 787 - 793 (2005) discloses that the preparation of porous polymeric materials with aligned porosity in the micrometre range, is of technological importance for a wide range of applications in organic electronics, micro fluidics, molecular filtration and biomaterials. It further demonstrates a generic method, based on directional freezing, for the preparation of aligned materials using polymers, nanoparticles or mixtures of these components as building blocks.

Reference may be made to patent application US 2003/0207975 A1, which discloses nanocomposite biomedical implants comprising a matrix and coated nanofillers wherein the coated nanofillers are dispersed in the matrix to form a composite. Various methods for making nanofillers and preparing the nanocomposite implants are also disclosed.

The nanoparticles of prior art possess varied properties. But there are no prior art that disclose scaffolds of nanoparticles where the nanoparticles are cross linked, so that the porous scaffolds are self standing. Further there are no prior art with regard to easy to use, generic methods that create the scaffold with control over pore sizes from a variety of commonly available materials. Also prior documents do not teach the cross linking of nanoporous scaffolds such that the scaffolds can be made self standing, and therefore can be applied widely in areas such as catalysis, electronic or electromagnetic devices, chromatography and such like.

Therefore, the objective is to form a self-standing scaffold with controllable porosity and have a precise control on the pore sizes and directionality. Long term goal seeks these scaffolds be used as cell growth substrates, as materials for solar cells, electrical and thermal insulators and also catalysts for several applications.

### SUMMARY OF THE INVENTION

Accordingly, the present invention provides a process for the preparation of a self standing scaffold or network of nanoparticles with controllably variable mesh size ranging between 500 nm to 1 mm, wherein said process comprises the steps of:
(i) dispersing the nano-particles with a size ranging between 5 and 500nm in the surfactant phase (50/50 composition of surfactant and water) at temperatures above the ordered phase-isotropic phase transition temperature to obtain surfactant-particle dispersion;
(ii) cooling the surfactant-particle dispersion of step (i) to a temperature such that a surfactant mesophase is formed;
(iii) optionally imposing flow on the mesophase-particle dispersion of step (ii) to obtain controllable orientation of the particle network and
(iv) cross linking the particles as obtained in step (ii) or (iii) to form the network.

In an embodiment of the present invention, the ordered phase isotropic phase transition temperature is the temperature at which the conversion occurs from ordered mesophase to disordered isotropic phase i.e. between 40-45 deg C.

In another embodiment of the present invention, the cross linking processes are selected from coating particles by absorbing a layer of cross linkable polymer, and preparing particles with cross linkable groups on their surface.

In another embodiment of the present invention, cross linkable polymer is selected from the group consisting of polyvinyl alcohol (PVA) and polyethyleneimine (PEI).

In another embodiment of the present invention, ratio of the cross linkable polymer and nanoparticle is ranging between 1:100 to 100:1 by weight.

In another embodiment of the present invention, cooling is done at the rate of 0.5-300°C/minute.

In yet another embodiment of the present invention, the cooling is done at 300°C/min resulting in mesh size of 500 nm.

In another embodiment of the present invention, the cooling is carried out at 0.5°C/min to obtain mesh size in the range of 200 µm.

Such scaffolds are useful in catalysis, electronic devices, electromagnetic devices, drug delivery, chromatography, tissue engineering and cell growth.

In still another embodiment of the invention, the process of the invention results in cross linking of anisotropic particles with specific relative orientation.

In yet another embodiment of the invention, the process of the invention results in the formation of directional pores by the imposition of flow prior to cross linking the particles.

In yet another embodiment of the invention, imposition of flow prior to cross-linking the particles results in the formation of directionally oriented pores.

### BRIEF DESCRIPTION OF THE FIGURE

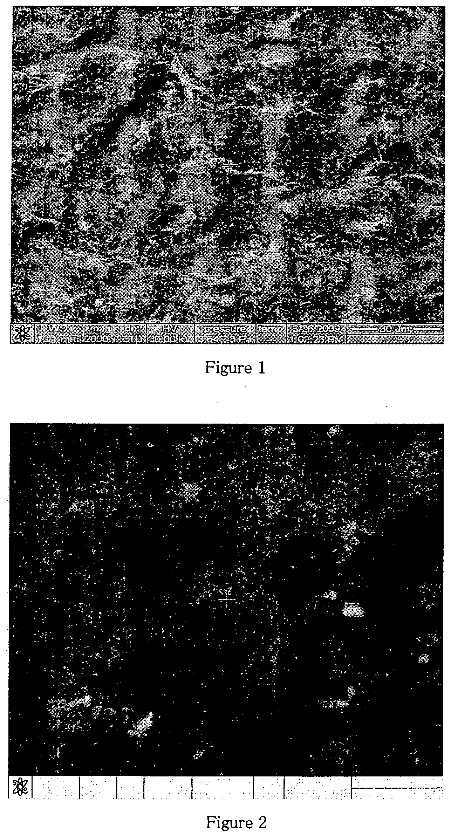

**Figure 1** depicts SEM of nano scaffold prepared by a process as in example 10. 12nm silica coated with polyethylene imine (MW = 2000 g/mol) with 100:1 ratio and cooled at 5°C/min. Surfactant is washed out after preparation and the material is dried before performing scanning electron microscopy (SEM).
**Figure 2** shows SEM of 12nm silica coated with polyethylene imine (MW = 2000 g/mol) with 100:1 ratio; cooled at 40°C/min. Surfactant is washed out after preparation and the material is dried before performing scanning electron microscopy (SEM).

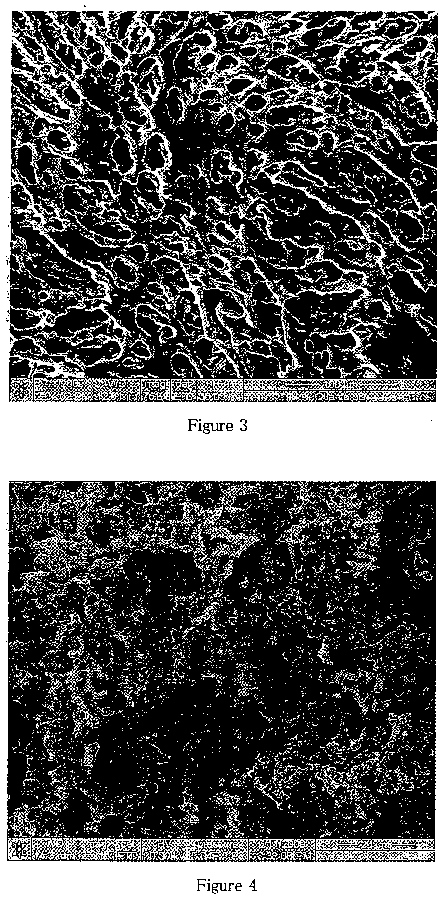

**Figure 3** SEM of 12nm silica particles coated with polyethylene imine (MW = 750,000 g/mol) with 100:1 ratio; cooled at 0.5°C/min and calcined in N₂ for 4hrs and subsequently in air for 6hrs is shown herein..
**Figure 4** depicts SEM of 500nm silica coated with polyethylene imine (MW = 750,000 g/mol) with 100:1 ratio; cooled at 5°C/min and calcined in N₂ for 4hrs and air for 6hrs.

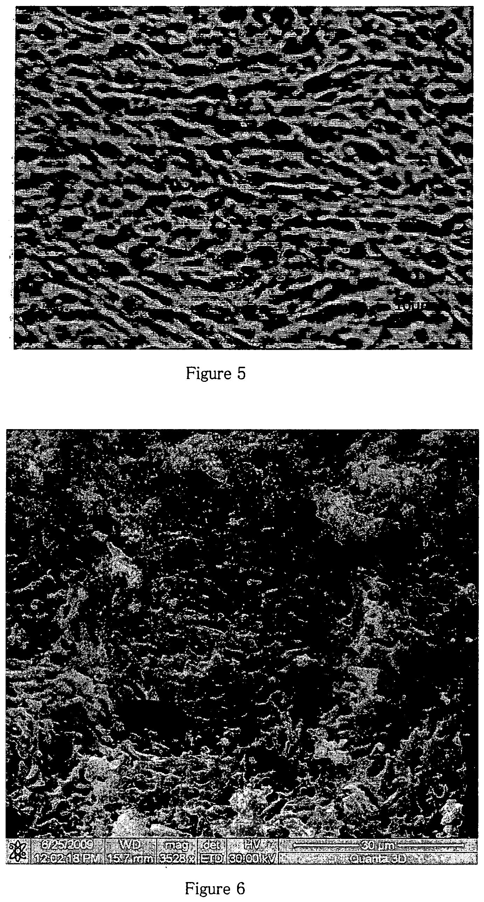

**Figure 5** shows optical micrograph of an oriented scaffold formed by shearing in a shear cell at 0.1 rad/s for 1 minute. The scaffold comprises of 15 nm silica particles coated with a polymer (polyethyleneimine) and subsequently crosslinked.
**Figure 6** is the SEM image of a calcined scaffold from assembly of 15 nm silica particles coated with a polymer (polyethyleneimine, with a molecular weight of 25000g/mol). The polymer was crosslinked and subsequently, the sample was calcined in air at 700°C for 6 hours, and subsequently in nitrogen at 700°C for 6 hours.

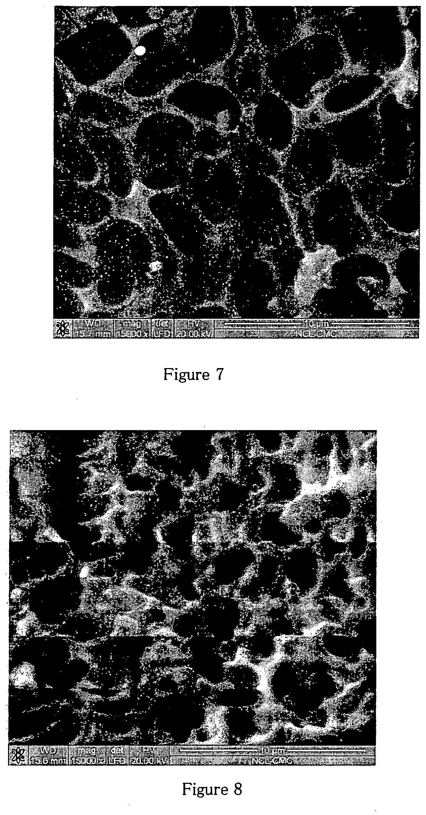

**Figure 7** depicts the control of pore size in scaffold by changing the cooling rate while the particles phase separate. The image on the left shows a 15 nm silica sample coated with polymer (2000g/mol PEI) and cooled from 50°C to 25°C at 10°C/min. The polymer is subsequently crosslinked using gluteraldehyde and the surfactant is washed out. The image on the right shows pores that are about two-fold larger. This sample is made exactly as the previous sample, except it is cooled from 50°C to 25°C at 5°C/min.
**Figure 8** illustrates optical micrograph of 2wt% Fe₃O₄ nanoparticles of size ^{∼}10nm self assembled in the form of network in the C₁₂E₉ -H₂O hexagonal phase. The network is crosslinked by coating the particles with Polyethyleneimine and subsequent crosslinking with glutaraldehyde.

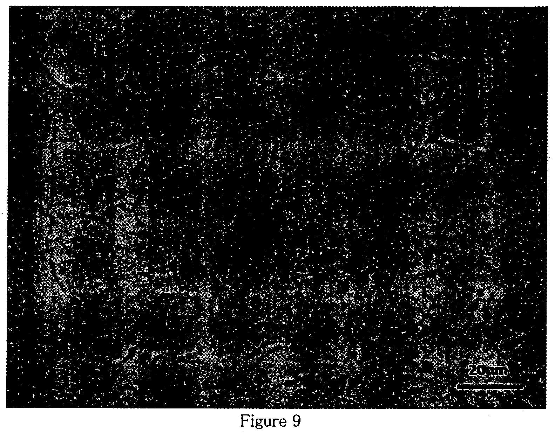

**Figure 9** Confocal micrograph of a 12nm silica particle scaffold tagged with a fluorescent dye is showed in this figure.. The scaffold was made by dispersing 12nm particles coated with Polyethylene imine (M.W. 2000g/mol) in a 1:1 C₁₂E₉:H₂O system at 50° C and then cooling it to 25° C at 5° C/min. The network thus formed was crosslinked with glutaraldehyde and the surfactant was subsequently removed by washing. The dye (Fluorescein, FITC) was tagged by overnight stirring of 50mg of scaffold with 0.2mg of FITC in a 50 ml ethanol solution. After the reaction the excess dye was then removed by centrifugation. The tagged porous scaffold can be seen in the figure clearly.

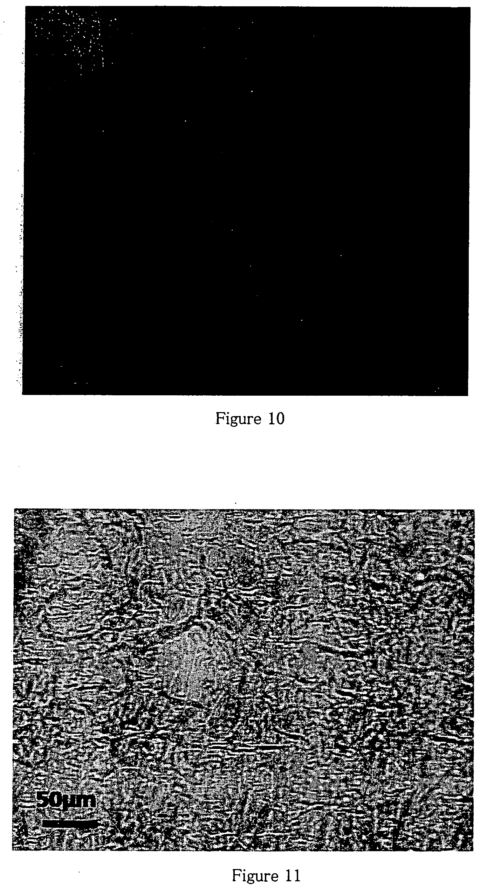

**Figure 10** Optical Micrograph of scaffold formed by 2% PNIPAM microgel (size 320nm)is depicted herein. The PNIPAM microgel particles were coated with Polyethyleneimine (M.W.25000 g/mol) and the pH of the coated particles was adjusted to 8. These microgel particles were then thrown in the 1:1 C₁₂E₉:H₂O mixture at 50° C and cooled to 25° C at 5° C/min. The microgel network thus formed was crosslinked with glutaraldehyde and subsequently the surfactant was washed with water.

### DETAILED DESCRIPTION OF THE INVENTION

The process of the present invention is for preparing a self standing network or scaffold of nanoparticles with independently controllable, variable network mesh size between 500nm and 1 mm, using nanoparticles, a surfactant capable of forming ordered structured phases and a cross linking agent, wherein the surfactant is washed off leaving the self standing scaffold.

The nanoparticles may be selected from metallic particles, inorganic particles, particles of organic compounds that are not soluble in the surfactant mesophase, polymeric compounds, semi conducting particles, magnetic nanoparticles and such like. The particles are of different geometries and can be isotropic (spherical) or anisotropic (including but not limited to, for example: rod-like, plate-like) or may be irregularly shaped.

The surfactant is capable of forming ordered, structured phase - hexagonal, lamellar, spongy, cubic network and such like, preferably hexagonal. The surfactant is CₙEₘ, wherein n>l, preferably > 10, m>1.

The self standing scaffold may comprise of a network of particulate strands with a controllably variable spacing and with a particle volume fraction of between 0.5 to 50%. The self standing scaffold exhibits porosity within the particulate strands, that are spaces between particles, controllably varied by using particles of different size, as well as porosity between strands, controllably varied by varying the particle volume fraction and/or by varying process parameters. The parameter to .be varied to control porosity is the cooling rate. Imposition of flow prior to cross-linking the particles results in the formation of directionally oriented pores.

The current invention describes the preparation of a self standing network of particles in a surfactant mesophase using silica or gold nanoparticles with a size between 5 and 500nm and a nonionic C₁₂E₉ hexagonal surfactant phase (50/50 composition of surfactant and water). Functional particles selected from, but not limited to quantum dots such as CdS, CdSe, ZnS and such like, ; particles with magnetic properties, ferromagnetic nanoparticles are used to form such networks. As the formation of the network is driven by expulsion of the particles from the mesophase, crystallizing or mesophase forming matrix are suitable to form the self standing scaffolds of the invention.

The process for preparing self standing network of nanoparticles of the invention with controllably variable mesh size comprises:
i. dispersing the particles in the surfactant at temperatures above the ordered phase-isotropic phase transition temperature;
ii. cooling the surfactant-particle dispersion to a temperature such that a surfactant mesophase is formed;
iii. optionally imposing flow on the mesophase-particle dispersion to obtain controllable orientation of the particle network; and
iv. cross linking the particles to obtain self-standing scaffold.

The rate of cooling determines the porosity of the self-standing scaffold. The rapid rate of cooling results in finer porosities, while slower rates results in coarser porosities. The rate of cooling ranges for 0.5 deg C/minute to 300 deg C/minute. As the cooling rate from the isotropic phase increases from 0.5°C /min to 5°C /min to 20°C /min; the size scale of the domain structure (and consequently of the particle network) decreases from about 25 µm to about 2-3 µm. Similarly, the rapid cooling rates results in an even finer network mesh of around 500nm. Thus, controlling the cooling rate is a facile way of engineering the mesh size of the particulate network of the self-standing scaffold.

Further, the cross linking of the particles is effected by cross linking processes, selected from, but not limited to, using coated particles by adsorbing a layer of cross linkable polymer, and preparing particles with crosslinkable groups on their surface, and such like.

The cross linking of the particles results in the self standing scaffolds. While scaffolds are described in prior art documents, self standing scaffolds prepared from any nano particle as described herein by a simple process applicable to the different types of particles as described and exemplified is hitherto undisclosed. The rate of cooling to control the porosity and the choice of cross linking processes to result in a simple process to prepare self standing scaffolds with independently controllable, variable network mesh size between 500nm and 1 mm is hitherto unknown.

The spatial organization of particles is a result of inter particle interactions mediated by the surfactant phase. Cooling the particle dispersion in the micellar surfactant phase into the hexagonal mesophase, results in local phase separation of the particles by expulsion from the mesophase to jam into a kinetically determined network structure.

The current invention utilized the particles of different sizes, 5nm up to 500nm; therefore, there is porosity within the particulate walls with a pore length scale comparable to the particle diameter, in addition to the "mesh" length scale. The material as made comprises of between 1 and 20% of the particles (weight per volume). This corresponds to a volume fraction of about 0.5 to 10%. A porosity of 90-99.5% is obtained with no change subsequent to cross linking, removal of solvent and such like. Drying after removal of the solvent optionally results in shrinkage of the material and optional partial collapse of the structure. The particle volume fraction between 0.5 to 50% is arrived at by consideration of amount of polymer used for the preparation and the porosity obtained in the scaffolds.

In an embodiment of the invention, cross linking of polymer coated particles are prepared. Silica particles are coated by adsorbing a layer of crosslinkable polymer on it, said cross linkable polymers are polyvinylalcohol, polyethyleneimine and such like. This is done in solution by preparing a dispersion of silica particles in water and adding a diluted solution of PVA or PEI to it while stirring/ sonicating method. The concentration of polymer is calculated to be between 1:100 and 100:1 (by weight) relative to the nanoparticle. The molecular weight of the polymer is controlled so as to prevent bridging between multiple particles, viz. one polymer chain sticking multiple particles together. Subsequent to completion of polymer coating, surfactant is added to the coated particle dispersion to form the particle networks. The polymer is optionally subsequently crosslinked using an agent such as gluteraldehyde. Subsequent to completion of cross linking, the surfactant/water is washed out using repeated washes with water and organic solvent to obtain a free-standing particle network.

Such scaffolds are used in catalysis, electronic devices, electromagnetic devices, drug delivery, chromatography, tissue engineering and cell growth.

### EXAMPLES

The following examples are given to illustrate the process of the present invention and should not be construed to limit the scope of the present invention.

### Example 1

Polyethylene imine (PEI) and polyvinyl alcohol (PVA) coated silica particles were prepared by mixing 5 ml of 25 wt % of silica particle aqueous dispersion with 1 ml of 100 mg/ml of PEI/PVA solution. Excess polymer is removed by centrifugation and washing with water steps. The coated particles are characterized by Zeta potential measurements The change in the surface charge of the particles from negative (around -30 mV) to positive (around +8 mV) occurs when polyethylene imine coats the particle.

### Reference Example A

Gold particles of size 50 nm (at a concentration of 0.1 M) were dispersed in water at 50 deg C, Nonaethylene glycol dodecyl ether (C₁₂E₉) was added such that the ratio of surfactant to water is 1:1 by weight, and cooled from 50°C to room temperature at a rate of 5 °C/minute. The gold particles organized to form a network and weld without any further external action, due to the large Hamaker constant of gold (large force of attraction between gold nanoparticles). The surfactant was then washed away with 1:1 water ethanol mixture. These washing steps were repeated 4 times and finally the sample was washed with acetone to leave the self-standing scaffold.

### Reference Example B

Rod-like gold nanoparticles (at concentrations of 0.1%, 0.5% and 0.85%, by weight) with a diameter of 20 nm and an aspect ratio of 3 were dispersed in water at 50 deg C, and C₁₂E₉ (water and C₁₂E₉ taken in equal parts) was added and cooled to room temperature at a rate of 5°C/minute. The gold nanoparticles were observed to weld due to the high force of attraction between gold. The nanoparticle network so generated has gold rods that are linked end-to-end as observed from Visible/near IRspectroscopy. With increase in the starting concentration of gold nanoparticles, the longitudinal plasmon peak in the UV-Vis spectrum shifts from 632 nm for 0.1% to 686 nm for 0.5% to 720 nm for 0.85% indicating end-to-end assembly of the rods.

### Example 2

1ml of 12 nm silica particles in a 30% (weight/volume) aqueous solution was mixed with an aqueous solution of polyvinyl alcohol (1:1 weight ratio of polyvinyl alcohol to silica; MW of polyvinyl alcohol = 9000 g/mol). These polymer covered particles were dispersed in water at 50 deg C, and C₁₂E₉ in an amount similar to water was added and cooled to room temperature at a rate of 300°C/minute. This was exposed to glutaraldehyde vapors for 24 hours and the polymer covered particles were cross linked to obtain the nanoparticle scaffold.

### Example 3

1ml of 12 nm silica particles in a 30% (weight/volume) aqueous solution was mixed with an aqueous solution of polyvinyl alcohol (1:100 weight ratio of polyvinyl alcohol to silica; MW of polyvinyl alcohol = 9000 g/mol). These polymer covered particles were dispersed in water at 50 deg C, and C₁₂E₉ in an amount similar to water was added and cooled to room temperature at a rate of 300°C/minute. This was exposed to glutaraldehyde vapors for 24 hours and the polymer covered particles were cross linked to obtain the nanoparticle scaffold.

### Example 4

1ml of 12 nm silica particles in a 30% (weight/volume) aqueous solution was mixed with an aqueous solution of polyvinyl alcohol (1:1 weight ratio of polyvinyl alcohol to silica; MW of polyvinyl alcohol = 9000 g/mol). These polymer covered particles were dispersed in water at 50 deg C, and C12E9 in an amount similar to water was added and cooled to room temperature at a rate of 20°C/minute. This was exposed to glutaraldehyde vapors for 24 hours and the polymer covered particles were cross linked to obtain the nanoparticle scaffold.

### Example 5

1ml of 12 nm silica particles in a 30% (weight/volume) aqueous solution was mixed with an aqueous solution of polyvinyl alcohol (1:1 weight ratio of polyvinyl alcohol to silica; MW of polyvinyl alcohol = 9000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 6

1ml of 12 nm silica particles in a 30% (weight/volume) aqueous solution was mixed with an aqueous solution of polyvinyl alcohol (1:1 weight ratio of polyvinyl alcohol to silica; MW of polyvinyl alcohol = 9000 g/ mol). These polymer covered particles were dispersed in water at 50 deg C, and C₁₂E₉ in an amount similar to water was added and cooled to room temperature at a rate of 0.5°C/minute. This was exposed to glutaraldehyde vapors for 24 hours and the polymer covered particles were cross linked to obtain the nanoparticle scaffold.

### Example 7

1ml of 12 nm silica particles in a 30% (weight/volume) aqueous solution was mixed with an aqueous solution of polyvinyl alcohol (1:100 weight ratio of polyvinyl alcohol to silica; MW of polyvinyl alcohol = 9000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 8

1ml of 12 nm silica particles in a 30% (weight/volume) aqueous solution was mixed with an aqueous solution of polyethylene imine (1:25 weight ratio of polyethylene imine to silica; MW of polyethylene imine = 9000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 9

1ml of 12 nm silica particles in a 30% (weight/volume) aqueous solution was mixed with an aqueous solution of polyethylene imine (1:100 weight ratio of polyethylene imine to silica; MW of polyethylene imine = 9000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 10

1ml of 12 nm silica particles in a 30% (weight/volume) aqueous solution was mixed with an aqueous solution of polyethylene imine (1:100 weight ratio of polyethylene imine to silica; MW of polyethylene imine = 1000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 11

1ml of 12 nm silica particles in a 30% (weight/volume) aqueous solution was mixed with an aqueous solution of polyethylene imine (1:100 weight ratio of polyethylene imine to silica; MW of polyethylene imine = 750000 g/mol). These polymer covered particles were dispersed in water at 50 deg C, and C₁₂E₉ in an amount similar to water was added and cooled to room temperature at a rate of 5°C/minute. This was exposed to glutaraldehyde vapors for 24 hours and the polymer covered particles were cross linked to obtain the nanoparticle scaffold.

### Example 12

Acrylamide coated silica particles were prepared, by dispersing 5wt% Silica of 40 nm in 100 ml Ethanol and overnight stirring with 2ml Aminopropyl Triethoxy silane (APTES) solution. The APTES coated particles were then covalently bonded to 0.01M Acrylic Acid solution leading to the formation of Acrylamide coated silica particles. These particles were used for photocrosslinking.

These were dispersed in water at 50 deg C, and C₁₂E₉ (water and C₁₂E₉ taken in equal parts) was added and cooled to room temperature at a rate of 5°C/minute. This composite was exposed to intense UV radiation resulting in cross linking of the surface groups to form a nanoparticulate network.

### Example 13

1ml of 12 nm silica particles in a 30% (weight/volume) aqueous solution was mixed with an aqueous solution of polyethylene imine (1: 100 weight ratio of polyethylene imine to silica; MW of polyethylene imine = 9000 g/mol). These polymer covered particles were dispersed in water at 50 deg C, and C₁₂E₉ (water and C₁₂E₉ taken in equal parts) was added and this was spun cast on a silicon substrate. This was exposed to gluteraldehyde to create a scaffold of cross-linked silica particles on a surface.

### Example 14

Polyvinyl alcohol covered (1g/sq m) cadmium selenide nanoparticles of 10 nm in size were dispersed in water at 50 deg C, and C₁₂E₉ (water and C₁₂E₉ taken in equal parts) was added and cooled to room temperature at a rate of 5°C/minute. This was exposed to gluteraldehyde vapors and the polymer covered particles were cross linked to obtain the nanoparticle scaffold. The surfactant was washed out to obtain a self standing CdSe scaffold. This scaffold was infiltrated with thiophene to create a self-standing scaffold of CdSe particles in thiophene.

### Example 15

1ml of 12 nm silica particles in a 30% (weight/volume) aqueous solution was mixed with an aqueous solution of polyvinyl alcohol (1 :1 weight ratio of polyvinyl alcohol to silica; MW of polyvinyl alcohol = 750000 g/mol). These polymer covered particles were dispersed in water at 50 deg C, and C₁₂E₉ in an amount similar to water was added and cooled to room temperature at a rate of 20°C/minute. This was exposed to glutaraldehyde vapors for 24 hours and the polymer covered particles were cross linked to obtain the nanoparticle scaffold.

### Example 16

1ml of 12 nm silica particles in a 30% (weight/volume) aqueous solution was mixed with an aqueous solution of polyvinyl alcohol (1:1 weight ratio of polyvinyl alcohol to silica; MW of polyvinyl alcohol = 1000 g/mol). These polymer covered particles were dispersed in water at 50 deg C, and C₁₂E₉ in an amount similar to water was added and cooled to room temperature at a rate of 20°C/minute. This was exposed to glutaraldehyde vapors for 24 hours and the polymer covered particles were cross linked to obtain the nanoparticle scaffold.

### ADVANTAGES OF THE INVENTION

i. The present process provides self-standing scaffold with controllable porosity and have a precise control on the pore sizes and directionality.
ii. The present process provides self-standing scaffold used growth substrates, as materials for solar cells, electrical and thermal insulators and also catalysts for several applications
iii. The present process provides cross linked nanoporous scaffolds such that the scaffolds are self standing, and
   therefore can be applied widely in areas such as catalysis, electronic or electromagnetic devices, chromatography and such like.

## Claims

1. A process for the preparation of a self standing scaffold or network of nanoparticles with controllably variable mesh size ranging between 500 nm to 1 mm, wherein said process comprises the steps of
i. dispersing the nano-particles with a size ranging between 5 and 500nm in the surfactant phase (50/50 composition of surfactant and water) at temperatures above the ordered phase-isotropic phase transition temperature to obtain surfactant-particle dispersion;
ii. cooling the surfactant-particle dispersion of step (i) to a temperature such that a surfactant mesophase is formed;
iii. optionally imposing flow on the mesophase-particle dispersion of step (ii) to obtain controllable orientation of the particle network and
iv. cross linking the particles as obtained in step (ii) or (iii) to form the network.

2. A process as claimed in claim 1, wherein the cross linking processes are selected from coating particles by absorbing a layer of cross linkable polymer, and preparing particles with cross linkable groups on their surface.

3. A process as claimed in claim 2, wherein cross linkable polymer is selected from the group consisting of polyvinyl alcohol (PVA) and polyethyleneimine (PEI).

4. A process as claimed in claim 2 or 3, wherein ratio of the cross linkable polymer and nanoparticle is ranging between 1:100 to 100:1 by weight.

5. A process as claimed in claim 1, wherein cooling is done at the rate of 0.5-300°C/minute.

## Patentansprüche

1. Verfahren zur Herstellung eines selbststehenden Gerüstes oder Netzwerkes von Nanopartikeln mit kontrollierbar veränderbarer Maschenweite, welche zwischen 500 nm und 1 mm beträgt, wobei das Verfahren die folgenden Schritte umfasst:
i. Verteilen der Nanopartikeln mit einer Abmessung zwischen 5 und 500 nm in der Tensidphase (Zusammensetzungsverhältnis von 50/50 von Tensid und Wasser), bei Temperaturen über der geordneten phasenisotropen Phasenübergangstemperatur, um eine Tensid-Partikel-Dispersion zu erhalten;
ii. Kühlen der Tensid-Partikel-Dispersion von Schritt (i) auf eine solche Temperatur, dass eine Tensid-Mesophase gebildet wird;
iii. optional, Auflegen einer Strömung auf der Mesophasen-Partikel-Dispersion von Schritt (ii), um eine kontrollierbare Ausrichtung des Partikelnetzwerkes zu erhalten, und
iv. Vernetzen der Partikeln, die aus Schritt (ii) oder (iii) erhalten werden, um das Netzwerk zu bilden.

2. Verfahren nach Anspruch 1, wobei die Vernetzungsverfahren ausgewählt sind aus: Beschichten der Partikeln durch Absorbieren einer Schicht von vernetzbarem Polymer, und Herstellen von Partikeln mit vernetzbaren Gruppen auf ihrer Oberfläche.

3. Verfahren nach Anspruch 2, wobei das vernetzbare Polymer ausgewählt ist aus der Gruppe, bestehend aus Polyvinylalkohol (PVA) und Polyethylenimin (PEI).

4. Verfahren nach Anspruch 2 oder 3, wobei das Verhältnis zwischen dem vernetzbaren Polymer und den Nanopartikeln im Bereich zwischen 1:100 und 100:1 Gewichtsanteile liegt.

5. Verfahren nach Anspruch 1, wobei das Kühlen mit einer Geschwindigkeit von 0,5-300°C/Minute ausgeführt wird.

## Revendications

1. Procédé pour la préparation d'un squelette ou réseau autoportant de nanoparticules avec une taille de maille variable de façon contrôlable s'inscrivant dans la plage entre 500 nm et 1 mm, dans lequel ledit procédé comprend les étapes consistant à :
i. disperser les nanoparticules présentant une taille qui s'inscrit dans la plage entre 5 et 500 nm dans la phase agent tensioactif (composition 50/50 d'agent tensioactif et d'eau) à des températures au-delà de la température de transition de phase ordonnée-phase isotrope de manière à obtenir une dispersion agent tensioactif-particules ;
ii. refroidir la dispersion agent tensioactif-particules de l'étape (i) jusqu'à une température qui est telle qu'une mésophase d'agent tensioactif est formée ;
iii. en option, imposer une circulation à la dispersion mésophase-particules de l'étape (ii) de manière à obtenir une orientation contrôlable du réseau de particules ; et
iv. réticuler les particules telles qu'obtenues au niveau de l'étape (ii) ou (iii) de manière à former le réseau.

2. Procédé tel que revendiqué selon la revendication 1, dans lequel les processus de réticulation sont sélectionnés parmi le revêtement de particules par absorption d'une couche de polymère réticulable et la préparation de particules avec des groupes réticulables sur leurs surfaces.

3. Procédé tel que revendiqué selon la revendication 2, dans lequel le polymère réticulable est sélectionné parmi le groupe qui est constitué par l'alcool polyvinylique (PVA) et le polyéthylèneimine (PEI).

4. Procédé tel que revendiqué selon la revendication 2 ou 3, dans lequel le rapport du polymère réticulable et des nanoparticules s'inscrit dans la plage entre 1:100 et 100:1 en poids.

5. Procédé tel que revendiqué selon la revendication 1, dans lequel le refroidissement est effectué à la vitesse de 0,5-300° C/minute.
